# EUROPÄISCHE PATENTANMELDUNG

(11) **EP 1 619 175 A1**
(43) Veröffentlichungstag der Anmeldung: **25.01.2006**
(21) Anmeldenummer: 04014642.5
(22) Anmeldetag: 22.06.2004
(51) Int. Cl.: C07C 17/14, C07C 17/20, C07C 201/12, C07C 205/11, C07C 22/08

(54) **Herstellung von fluorierten Alkylaromaten**

(71) Anmelder: Solvay Fluor GmbH, 30173 Hannover (DE)
(72) Erfinder: Braun, Max Dr., 30900 Wedemark (DE)
(74) Vertreter: Fischer, Reiner

(57) **Zusammenfassung**

Alkylbenzole mit verzweigter, poly- oder perhalogenierter Alkylgruppe können aus den entsprechenden Alkylverbindungen durch Chlorierung und anschließende Fluorierung hergestellt werden. Gewünschtenfalls kann man von kernsubstituierten Derivaten ausgehen oder den aromatischen Kern nach der Halogenierung substituieren. Das Verfahren eignet sich besonders für die Herstellung von Perfluorisopropylcumol und seinen Derivaten.

## Beschreibung

Die Erfindung bezieht sich auf ein Verfahren zur Herstellung von Benzolverbindungen, die durch eine gegebenenfalls verzweigte, polyfluor-, perfluor- oder perhalogenierte Alkylgruppe sowie gewünschtenfalls am Benzolring zusätzlich durch ein oder mehrere Halogenatome und/oder die Nitrogruppe substituiert sind, wobei mindestens ein Halogenatom an der verzweigten Alkylgruppe in Form eines Fluoratoms vorliegt.

Derartige Verbindungen sind Bausteine in der chemischen Synthese. So ist aus der US 6,717,013 bekannt, siehe dort Spalte 1, dass Perfluoralkylanilinverbindungen nützliche Zwischenprodukte für die Synthese von Agrochemikalien, Pharmazeutika, oberflächenaktiven Substanzen und anderen Mitteln sind. Die Herstellung derartiger Anilinverbindungen wird dort auch beschrieben, z.B. durch Perfluoralkylierung ausgehend von Perfluoralkylhalogeniden und Kupferpulver, durch Anlagerung von Perfluoralkenen an Fluornitrobenzol oder, entsprechend der dort beschriebenen Erfindung, durch Umsetzung eines Anilins mit einer Perfluoralkyliodid-Verbindung.

Aufgabe der vorliegenden Erfindung ist es, alternative Synthesewege zur Herstellung solcher Verbindungen bzw. von Vorläufern solcher Verbindungen anzugeben. Diese Aufgabe wird durch das in den Ansprüchen angegebene Verfahren gelöst.

Die Erfindung beruht auf der Erkenntnis, dass man von den entsprechenden Alkylverbindungen ausgeht und die Alkylgruppe halogeniert, insbesondere perfluoriert. Dies kann direkt über eine Fluorierung mit elementarem Fluor geschehen, oder man führt zunächst eine Chlorierung mit anschließendem Chlor-Fluor-Austausch durch.

Das erfindungsgemäße Verfahren zur Herstellung von Verbindungen mit einem Benzolring, der durch eine Polyfluor-, Perfluor- oder perfluorchlorsubstituierte Alkylgruppe mit 1 bis 8 C-Atomen substituiert ist und gegebenenfalls zusätzlich durch ein oder mehrere Halogenatome und/oder durch die Nitrogruppe substituiert sein kann, mit der Maßgabe, dass mindestens 1 Halogenatom an der verzweigten Alkylgruppe ein Fluoratom ist, sieht vor, dass man von einer Verbindung ausgeht, die die entsprechende nicht mit Halogen substituierte Alkylgruppe aufweist und man
a) in einem ersten Schritt eine Chlorierung der Alkylgruppe und anschließend einen Chlor-Fluor-Austausch vornimmt oder
b) eine Fluorierung der Alkylgruppe mit elementarem Fluor vornimmt,
und, sofern der Benzolring durch eine oder mehrere Halogenatome und/oder die Nitrogruppe substituiert sein soll, man entweder von einer bereits derartig substituierten Ausgangsverbindung ausgeht oder man den oder die Substituenten am Benzolring nach der Fluorierung einführt.

"Polyfluorsubstituiert" bedeutet, dass mindestens die Hälfte der Wasserstoffatome der Alkylgruppe durch Fluoratome ersetzt sind. "Perfluoriert" bedeutet, dass sämtliche Wasserstoffatome der Alkylgruppe durch Fluoratome ersetzt sind. "Perhalogeniert" bedeutet, dass sämtliche Wasserstoffatome durch Chlor und mindestens ein Fluoratom ersetzt sind.

Bevorzugt sind Verbindungen mit Alkylgruppen, die in α-Stellung verzweigt sind. Besonders bevorzugt sind solche mit einer verzweigten C3-C5-Alkylgruppe, insbesondere bevorzugt sind Verbindungen mit einer Isopropylgruppe.

Sofern man eine Fluorierung mit elementarem Fluor bewirkt, setzt man zweckmäßig Fluor ein, das mit Inertgas verdünnt ist. Gut geeignet ist Stickstoff als Inertgas. Die Fluorierung wird bei niedriger Temperatur, gegebenenfalls in einem Lösungsmittel, z.B. in einem perfluorierten Kohlenstoff, durchgeführt. Man fluoriert so lange, bis der gewünschte Fluorierungsgrad erreicht ist.

Eine Alternative zum Verfahren unter Verwendung von elementarem Fluor ist die Chlorierung mit anschließender Fluorierung unter Chlor-Fluor-Austausch. Dabei wird die verzweigte Alkylgruppe zunächst chloriert. Diese Chlorierung wird mit elementarem Chlor vorgenommen und kann thermisch, photochemisch oder thermisch mit Unterstützung durch einen Radikalinitiator, z.B. Benzoylperoxid oder Azobisisobutyronitril, bewirkt werden. Die Chlorierung wird solange durchgeführt, bis der gewünschte Chlorierungsgrad erreicht ist. Bevorzugt wird mindestens die Hälfte aller Wasserstoffatome an der Alkylgruppe gegen Chlor ausgetauscht, bevorzugt alle Wasserstoffatome, so dass eine Perchloralkylgruppe vorliegt.

Sofern man eine photochemische Chlorierung vornimmt, kann es zweckmäßig sein, einen Wellenbereich vorzusehen, bei welchem das Chlor zwar Strahlung absorbiert, nicht aber die Ausgangsverbindung oder auch das Produkt.

Die resultierende chlorierte Zwischenverbindung kann durch übliche Methoden isoliert durch Extraktion oder Destillation und gewünschtenfalls durch weitere Destillations- oder Extraktionsschritte gereinigt werden. Alternativ kann man die Reaktionsmischung aus der Chlorierung in die Fluorierungsreaktion einsetzen.

Die Fluorierung wird mit üblichen Mitteln zum Chlor-Fluor-Austausch vorgenommen. Üblicherweise verwendet man HF. Die Fluorierung kann ohne Katalysator oder mit Katalysator durchgeführt werden. Um einen höheren Fluorierungsgrad zu erreichen, verwendet man zweckmäßig einen Katalysator. Als Beispiele für Katalysatoren sollen erwähnt werden: Antimonhalogenide mit 3- oder fünfwertigem Antimon oder deren Gemische (derartige Verbindungen liegen meist überwiegend als Fluorverbindungen vor; siehe z.B. US 5315046); TiCl₄ oder SnCl₄ oder deren Gemische, siehe z.B. die US 5894075; Hydrofluorid-Adukte von Aminen, wie sie z.B. für die Fluorierung anderer Verbindungen beutzt werden, siehe die WO 00/32549; TiF₄, TiCl₄, SnCl₄ oder SnF₄ oder die entsprechenden gemischten Chlor-Fluor-Verbindungen.

Bei zumindest den Antimonhalogenid-Katalysatoren kann die Fähigkeit, auch höhere Fluorierungsgrade zu katalysieren, gesteuert werden. Je höher der Anteil an Fluoratomen im Antimonhalogenid-Katalysator und auch an HF in der Reaktionsmischung, desto aktiver ist der Katalysator.

Die nach dem erfindungsgemäßen Verfahren herstellbaren Verbindungen können am Benzolring durch ein oder mehrere Halogenatome und/oder die Nitrogruppe substituiert sein.

Sofern man Verbindungen herstellen möchte, die Halogenatome oder die Nitrogruppe in p- oder o-Position zum Alkylrest aufweisen sollen, empfiehlt es sich, entweder schon von derartig substituierten Verbindungen auszugehen oder diese Halogenatome bzw. die Nitrogruppe nach der Halogenierung des Alkylrestes einzuführen, da die halogenierte Alkylgruppe, besonders wenn sie fluoriert ist, einen Effekt aufweist, der die Substitution am Benzolring in die o- bzw. p-Position dirigiert.

Sofern man Verbindungen herstellen möchte, die das oder die Halogenatome bzw. die Nitrogruppe in m-Position aufweisen sollen, empfiehlt es sich, von bereits derartig substituierten Verbindungen auszugehen, weil die Kernsubstitution nach der Halogenierung des Alkylrestes bevorzugt in die o- bzw. p-Position gedrängt würde.

Das erfindungsgemäße Verfahren eignet sich besonders zur Herstellung von Cumol, das in der Isopropylgruppe perfluoriert ist und gewünschtenfalls am Benzolring durch eine Nitrogruppe oder ein Halogenatom, vorzugsweise in p-Position, substituiert ist. Zur Herstellung einer solchen Verbindung geht man zweckmäßig aus von der gewünschtenfalls am Kern bereits substituierten Cumol-Verbindung. Diese wird entweder mit elementarem Fluor direkt fluoriert, oder man stellt zunächst die Perchlorisopropylverbindung her, die dann unter Chlor-Fluor-Austausch unter Einsatz von HF in Anwesenheit eines der oben genannten Fluorierungskatalysatoren in die entsprechende Perfluorisopropylverbindung umgewandelt wird.

Das erfindungsgemäße Verfahren eignet sich besonders zur Herstellung von Verbindungen mit verzweigter Perfluoralkylgruppe und p-Nitro- oder o-Nitrosubstitution am Benzolring. Man kann dann o-Nitrocumol oder p-Nitrocumol (dies sind bekannte Verbindungen, CAS-Nummer 6526-72-3 bzw. 1817-47-6) einsetzen, diese wie oben beschrieben direkt oder über die perchlorierten Verbindungen fluorieren. Anschließend kann nach bekannten Methoden eine Reduktion der Nitrogruppe zur Aminogruppe vorgenommen werden, beispielsweise mittels Wasserstoff über Platin.

Verbindungen, die nach dem erfindungsgemäßen Verfahren hergestellt werden können, sind z.B. 4-Heptafluoroisopropylnitrobenzol und daraus durch Reduktion der Nitrogruppe das entsprechende 4-Heptafluoroisopropylanilin; 2-Fluoro- bzw. 3-Fluoro-4-heptafluoroisopropylnitrobenzol und daraus durch Reduktion der Nitrogruppe das entsprechende Anilin; 3-Chlor-4-Heptafluorisopropylnitrobenzol und daraus durch Reduktion das entsprechende Anilin; anstelle der genannten 4-Heptafluorisopropylverbindungen die entsprechenden Perfluormethyl-, Perfluorethyl-, Perfluor-n-propyl-, Perfluorbutylverbindungen.

Die entsprechend dem erfindungsgemäßen Verfahren herstellbaren Verbindungen eignen sich als Zwischenprodukte in der chemischen Synthese von Agrochemikalien, Pharmazeutika und anderen Mitteln, wie im US-Patent 6,717,013 beschrieben.

## Patentansprüche

1. Verfahren zur Herstellung von Verbindungen mit einem Benzolring, der durch eine polyfluor-, oder perfluor- oder perhalogensubstituierte Alkylgruppe mit 1 bis 8 C-Atomen substituiert ist und gegebenenfalls zusätzlich durch ein oder mehrere Halogenatome und/oder durch die Nitrogruppe substituiert sein kann, mit der Maßgabe, dass mindestens 1 Halogenatom an der verzweigten Alkylgruppe ein Fluoratom ist, wobei man von einer Verbindung ausgeht, die die entsprechende nicht mit Halogen substituierte Alkylgruppe aufweist und man
a) in einem ersten Schritt eine Chlorierung und anschließend einen Chlor-Fluor-Austausch vornimmt oder
b) eine Fluorierung mit elementarem Fluor vornimmt,
und, sofern der Benzolring durch eine oder mehrere Halogenatome und/oder die Nitrogruppe substituiert sein soll, man entweder von einer bereits derartig substituierten Ausgangsverbindung ausgeht oder man den oder die Substituenten am Benzolring nach der Fluorierung einführt.

2. Verfahren nach Anspruch 1, **dadurch gekennzeichnet, dass** die Alkylgruppe der Ausgangsverbindung in α-Stellung verzweigt ist.

3. Verfahren nach Anspruch 2, **dadurch gekennzeichnet, dass** die Alkylgruppe eine in α-Stellung verzweigte C3-C5-Alkylgruppe, vorzugsweise eine Isopropylgruppe ist.

4. Verfahren nach Anspruch 1, **dadurch gekennzeichnet, dass** man Perfluorisopropylbenzol herstellt, das am Benzolring gewünschtenfalls durch ein oder mehrere Halogenatome und/oder durch eine Nitrogruppe am Ring substituiert sein kann.

5. Verfahren nach Anspruch 4, **dadurch gekennzeichnet, dass** man die Isopropylgruppe von lsopropylbenzol, das am Benzolring gewünschtenfalls durch ein oder mehrere Halogenatome und/oder durch eine Nitrogruppe am Ring substituiert sein kann, mit elementarem Chlor perchloriert, wobei die Chlorierung thermisch, photochemisch oder thermisch, unterstützt durch einen Radikalinitiator durchgeführt wird.

6. Verfahren nach Anspruch 1, **dadurch gekennzeichnet, dass** man von einer bereits am Benzolring durch ein Halogenatom und/oder eine Nitrogruppe substituierten Verbindung ausgeht und die Chlorierung bzw. Fluorierung vornimmt, sofern man eine Substitution in meta-Stellung des Halogenatoms oder der Nitrogruppe zur Alkylgruppe wünscht, oder dass man den Benzolring nach der Chlorierung bzw. Fluorierung mit Halogen oder der Nitrogruppe substituiert, sofern man eine Substitution in der ortho- oder para-Position wünscht.

7. Verfahren nach Anspruch 1, **dadurch gekennzeichnet, dass** man die Fluorierung katalytisch durch Chlor-Fluor-Austausch mittels HF in der Flüssigphase durchführt.

8. Verfahren nach Anspruch 1, **dadurch gekennzeichnet, dass** man Verbindungen herstellt, die in o- oder p-Position zur halogenierten Alkylgruppe durch eine Nitrogruppe substituiert sind.

9. Verfahren nach Anspruch 8, **dadurch gekennzeichnet, dass** man die Nitrogruppe zu einer NH₂-Gruppe reduziert.

10. Verfahren nach Anspruch 1, **dadurch gekennzeichnet, dass** man Verbindungen herstellt, deren C3-C8-Alkylgruppe perfluoriert ist.
